# FASCICULE DE BREVET EUROPEEN

(11) **EP 0 587 855 B1**
(45) Date de publication et mention de la délivrance du brevet: **07.01.1998**
(21) Numéro de dépôt: 93907918.2
(22) Date de dépôt: 31.03.1993
(51) Int. Cl.: A61K 7/155, A61K 7/00

(54) **COMPOSITION A ACTION DESTABILISATRICE ET ATROPHIANTE DU POIL**
HAARDESTABILISIERENDE UND -ATROPHIERENDE ZUSAMMENSETZUNG
COMPOSITION HAVING A HAIR DESTABILIZING AND ATROPHYING ACTION

(30) Priorité: 01.04.1992 FR 9203960
(43) Date de publication de la demande: 23.03.1994
(73) Titulaire: LABORATOIRE GAM S.A., 93200 Saint Denis (FR)
(72) Inventeur: MAUGER, Charles, F-94100 Saint-Maur-Des-Fosses (FR)
(74) Mandataire: Peaucelle, Chantal
(86) Numéro de dépôt international: FR9300327
(87) Numéro de publication internationale: WO9319726

(56) Documents cités:
- EP-A- 0 096 521
- EP-A- 0 307 129
- FR-A- 552 508
- FR-A- 1 396 582
- FR-A- 2 585 566
- FR-A- 2 606 278
- US-A- 1 474 512
- US-D- 461 352
- CHEMICAL ABSTRACTS, vol. 93, no. 26, 1980, Columbus, Ohio, US; abstract no. 245276t, O. KRAJAKOVA 'depilation agent' page 374 ;
- CHEMICAL ABSTRACTS, vol. 110, no. 22 Columbus, Ohio, US; abstract no. 198956, R. OWADA ET AL. 'depilatories containing water-soluble polymers and alcohol-soluble polymers'
- DATABASE WPIL Section Ch, Week 9013, Derwent Publications Ltd., London, GB; Class B04, AN 90096013

## Description

La présente invention concerne une méthode de traitement cosmétique et une composition pour l'épilation définitive du corps humain, particulièrement destinée aux professions de l'esthétique.

On utilise actuellement, dans les instituts de beauté, des crèmes ou lotions post-épilatoires, qui n'ont pour seul effet que de retarder la repousse du poil. Si ces produits ont un effet de ralentissement et parfois même de diminution de la repousse; ils n'ont pas d'action vraiment définitive sur le poil et sont exclusivement utilisés après l'épilation.

La composition, selon l'invention, s'utilise avant l'épilation et a pour action de déstabiliser le poil à sa racine et d'en atrophier tous les éléments de repousse, jusqu'à sa complète disparition. Cette composition, selon l'invention, se présente sous la forme d'un gel, applicable directement sur la zone à épiler.

L'invention vise une méthode de traitement cosmétique en vue d'une épilation définitive, caractérisée par l'application, avant épilation, sur la zone à épiler, d'une composition sous forme de gel de contact renfermant des huiles essentielles et à action déstabilisatrice et atrophiante du poil, puis par un chauffage de cette composition pour la porter à une température de 25 à 50°C.

Selon une première caractéristique, ce gel est composé d'un mélange d'huiles essentielles dans un support d'huile végétale. Selon une autre caractéristique, la composition utilisée renferme en outre du camphre, de l'alcool et un calmant ou un adoucissant, d'origine végétale, l'ensemble étant incorporé à un gel de type gel de contact.

Selon encore une autre caractéristique, les huiles utilisées pour réaliser cette préparation, seront prises, de préférence, parmi les suivantes : Bouleau, Citron, Cyprès, Géranium, Lavande, Pin, Verveine. On utilise ces huiles dans les proportions suivantes :
- Bouleau de: 5 à 30 gouttes
soit 0,11 à 0,64 ml (47 gouttes par ml),
- Citron de: 15 à 120 gouttes
soit 0,41 à 3,24 ml (37 gouttes par ml),
- Cyprès de: 5 à 30 gouttes
soit 0,12 à 0,70 ml (43 gouttes par ml),
- Géranium de: 5 à 50 gouttes
soit 0,15 à 1,25 ml (40 gouttes par ml),
- Lavande de: 5 à 120 gouttes
soit 0,12 à 2,86 ml (42 gouttes par ml),
- Pin de: 5 à 30 gouttes
soit 0,11 à 0,65 ml (46 gouttes par ml),
- Verveine de: 10 à 80 gouttes
soit 0,23 à 1,86 ml (43 gouttes par ml),
On ajoute à ces huiles de 10 à 200 gouttes de camphre, soit 0,22 à 4,44 ml (45 gouttes par ml).

Ce premier mélange est ajouté à une huile végétale, par exemple Amande douce, dans une proportion de 2 ml à 20 ml. L'ensemble est émulsionné avec de l'alcool à 90 degrés; de 2,5 ml à 30 ml. On introduit ensuite dans le mélange, un calmant ou un adoucissant d'origine végétale, par exemple de l'azulène de 2 ml à 25 ml. Cette émulsion est enfin incorporée à une quantité de gel de contact, allant de 5 ml à 150 ml.

Dans une solution préférentielle, et à titre d'exemple, il a été retenu la formule suivante :

| | |
|---|---|
| Bouleau | 20 gouttes |
| Citron | 60 gouttes |
| Cyprès | 20 gouttes |
| Géranium | 20 gouttes |
| Lavande | 60 gouttes |
| Pin | 20 gouttes |
| Verveine | 40 gouttes |
| Camphre | 60 gouttes |
| Huile d'amande douce | 5 ml |
| Alcool à 90 degrés | 5 ml |
| Azulène | 2 ml |

Composition du gel de contact :

| | |
|---|---|
| Hydroéthylcellulose | 2,5 |
| Propylène glycol | 4 |
| P. Hydroxybenzoates de Méthyle et propyle | 0,15 |
| Sulfate de sodium | 1 |
| Bleu patente V | 2 ppm |
| Eau purifiée Q.S.P. | 100 g |

L'ensemble doit être brassé fortement pour obtenir un gel parfaitement homogène.

L'invention vise une composition à action déstabilisatrice et atrophiante du poil caractérisée en ce qu'elle est utilisable avant épilation et se présente sous forme d'une composition de gel telle qu'appliquée dans la méthode définie définie ci-dessus.

Le gel est appliqué directement sur la peau de la zone à épiler. Il s'est révélé quasiment indispensable de dynamiser l'action du gel par un apport thermique constant, d'une durée pouvant varier de 5 à 40 minutes.

L'invention vise donc l'ensemble comportant ladite composition de gel et un dispositif d'apport thermique constant.

Cet apport pourra s'effectuer de différentes manières; lampes infrarouges, air chaud pulsé; mais on préférera à ces méthodes, plus ou moins régulières dans la stabilité et la répartition de la chaleur, l'emploi d'un appareil à régulation thermique constante et contrôlée, qui offre une température d'application de 25 à 50 degrés Celsius.

Pour une utilisation optimale de la composition selon l'invention, cet appareil est constitué d'au moins trois générateurs thermiques réglables et équipés, chacun, d'une sortie indépendante, qui alimente des éléments chauffants de forme anatomique adaptée aux différentes zones du corps à épiler, et dont l'intensité de chauffage peut être réglée, en permanence, à partir du générateur thermique, ceci en fonction de la zone épilée et de la sensibilité de la personne traitée.

Les éléments chauffants sont constitués d'un ou plusieurs circuits de fil chauffant de longueur et résistance variables; la résistance idéale se situant entre 2 et 10 ohms/mètre, pour un ampérage de 2 à 10 ampères dans une tension allant de 5 à 38 volts.

Le fil chauffant des éléments est contenu dans une enveloppe textile résistant au feu, laquelle est habillée de skaï ou de plastique souple pour un nettoyage plus aisé. Le fil chauffant peut être aussi être contenu dans des formes moulées et souples en silicone ou dérivés.

A titre d'exemple, la stimulation idéale du gel est obtenue après une application de 30 mns sous une température variant au maximum de 36 à 39 degrés Celsius.

La préparation, selon l'invention, est particulièrement destinée à l'épilation définitive du corps humain, pratiquée en institut de beauté.

## Revendications

1. Méthode de traitement cosmétique en vue d'une épilation définitive, caractérisée par l'application, avant épilation, sur la zone à épiler, d'une composition à action déstabilisatrice et atrophiante du poil, sous forme de gel de contact renfermant des huiles essentielles, puis par un chauffage de cette composition pour la porter à une température de 25 à 50°C.

2. Méthode selon la revendication 1, caractérisée en ce que la composition utilisée est élaborée à partir d'un mélange d'huiles essentielles et d'huile végétale.

3. Méthode selon la revendication 2, caractérisée en ce que la composition utilisée renferme, en outre, du camphre, de l'alcool, un calmant ou adoucissant d'origine végétale.

4. Méthode selon l'une des revendications 1 ou 3, caractérisée en ce que les huiles essentielles sont choisies parmi l'huile de bouleau, de citron, de cyprès, de géranium, de lavande, de pin et de verveine.

5. Méthode selon l'une des revendications 2 à 3, caractérisée en ce que l'huile végétale est de l'huile d'amande douce.

6. Méthode selon l'une des revendications 3 à 5, caractérisée en ce que le calmant ou adoucissant est de l'azulène.

7. Méthode selon l'une des revendications 1 à 6, caractérisée en ce que le gel élaboré à partir d'hydroxyéthylcellulose, de propylène glycol, de p-hydroxybenzoates de méthyle et de propyle, et de sulfate de sodium.

8. Méthode selon l'une des revendications 1 à 7, caractérisée en ce que la composition renferme un mélange des huiles essentielles selon la revendication 4, selon les proportions suivantes :
Huiles de bouleau 5 à 30 gouttes,
soit 0,11 à 0,64 ml (47 gouttes par ml)
Huile de citron 15 à 120 gouttes,
soit 0,41 à 3,24 ml (37 gouttes par ml)
Huile de cyprès 5 à 30 gouttes,
soit 0,12 à 0,70 ml (43 gouttes par ml)
Huile de géranium 5 à 50 gouttes,
soit 0,15 à 1,25 ml (40 gouttes par ml)
Huile de lavande 5 à 120 gouttes,
soit 0,12 à 2,86 ml (42 gouttes par ml)
Huile de pin 5 à 30 gouttes,
soit 0,11 à 0,65 ml (46 gouttes par ml)
Huile de verveine 10 à 80 gouttes,
soit 0,23 à 1,86 ml (43 gouttes par ml)

9. Méthode selon la revendication 8, caractérisée en ce qu'elle renferme en outre du camphre à raison de 10 à 200 gouttes, soit 0,22 à 4,44 ml (45 gouttes par ml).

10. Méthode selon la revendication 8 ou 9, caractérisée en ce qu'elle renferme en outre de l'huile végétale à raison de 2 à 20 ml.

11. Méthode selon l'une des revendications 8 à 10, caractérisée en ce qu'elle renferme en outre de l'alcool à 90°, à raison de 2,5 à 30 ml.

12. Méthode selon l'une des revendications 8 à 11, caractérisée en ce qu'elle renferme en outre un calmant ou adoucissant à raison de 2 à 25 ml.

13. Méthode selon l'une des revendications 8 à 12, caractérisée en ce qu'elle comprend un gel répondant à la composition suivante :
| | |
|---|---|
| Hydroéthylcellulose | 2,5 g |
| Propylène glycol | 4 g |
| P. Hydroxybenzoates de méthyle et propyle | 0,15 g |
| Sulfate de sodium | 1 g |
| Bleu patent V | 2 ppm |
| Eau purifiée QSP | 100 g |

14. Méthode selon l'une des revendications 1 à 13, caractérisée en ce que le chauffage est réalisé à l'aide d'un appareil comprenant des éléments chauffants de forme adaptée à l'anatomie des zones à épiler, dans une enveloppe revêtue d'une matière plastique souple.

15. Composition à action déstabilisatrice et atrophiante du poil, caractérisée en ce qu'elle est utilisable avant épilation et se présente sous forme d'une composition telle qu'appliquée dans la méthode définie dans l'une quelconque des revendications 1 à 13.

16. Procédé d'élaboration d'une composition selon la revendication 15, caractérisé en ce qu'il comprend l'addition de camphre à un mélange d'huiles essentielles, suivie de l'addition du mélange obtenu à une huile végétale, puis de l'émulsion de l'ensemble avec de l'alcool à 90°, de l'addition d'un calmant ou adoucissant, puis de l'incorporation de l'émulsion au gel, avec brassage pour obtenir une composition homogène.

17. Ensemble pour déstabiliser, avant épilation, les poils à leur racine et atrophier les éléments de repousse, caractérisé en ce qu'il comporte une composition selon la revendication 15 et un dispositif ayant des élements chauffants de forme adaptée à l'anatomie des zones à épiler, permettant de dynamiser l'action de cette composition en la portant, après application sur la peau, à une température de 25 à 50°C.

## Claims

1. A method of comestic treatment for permanent depilation, comprising applying on the area to be depilated, before depilation, a hair destabilizing and atrophying composition, in the form of a contact gel containing essential oils and then heating said composition to bring it to a temperature of 25 to 50°C.

2. The method according to claim 1, wherein the used composition is elaborated from a mixture of essential oils and vegetal oil.

3. The method according to claim 2, wherein the used composition further contains camphor, alcohol, a sedative or softener of vegetable origin.

4. The method according to anyone of claims 1 or 3, wherein the essential oils are selected from among birch, lemon, cypress, geranium, lavender, pine and vervain oil.

5. The method according to anyone of claims 2 to 3, wherein the vegetal oil is sweet almond oil.

6. The method according to anyone of claims 3 to 5, wherein the sedative or softener is azulene.

7. The method according to anyone of claims 1 to 6, wherein the gel is elaborated from hydroxyethylcellulose, propyleneglycol, methyl and propyl p-hydroxybenzoates, and sodium sulphate.

8. The method according to anyone of claims 1 to 7, wherein the composition contains a mixture of the essential oils according to claim 4, according to the following proportions:
birch oil 5 to 30 drops
i.e. 0.11 to 0.64 ml (47 drops per ml)
lemon oil 15 to 120 drops
i.e. 0.41 to 3.24 ml (37 drops per ml)
cypress oil 5 to 30 drops
i.e. 0.12 to 0.70 (43 drops per ml)
geranium oil 5 to 50 drops
i.e. 0.15 to 1.25 ml (40 drops per ml)
lavender oil 5 to 120 drops
i.e. 0.12 to 2.86 ml (42 drops per ml)
pine oil 5 à 30 drops
i.e. 0.11 to 0.65 ml (46 drops per ml)
vervain oil to 10 to 80 drops i.e. 0.23 to 1.86 ml (43 drops per ml)

9. The method according to claim 8, characterized in that it further contains camphor on the basis of 10 to 200 drops, i.e. 0.22 to 4.44 ml (45 drops per ml).

10. The method according to claim 8 or 9, characterized in that it further contains vegetal oil on the basis of 2 to 20 ml.

11. The method according to anyone of claims 8 to 10, characterized in that it further contains alcohol at 90 degrees, on the basis of 2.5 to 30 ml.

12. The method according to anyone of claims 8 to 11, characterized in that it further contains a sedative or softener on the basis of 2 to 25 ml.

13. The method according to anyone of claims 8 to 12, characterized in that it contains a gel having the following composition:
| | |
|---|---|
| Hydroethylcellulose | 2.5 g |
| Propyleneglycol | 4 g |
| Methyl and propyl p-hydroxybenzoates | 0.15 g |
| Sodium sulphate | 1 g |
| Blue patent V | 2 ppm |
| Purified water QSP | 100 g |

14. The method according to anyone of claims 1 to 13, wherein the heating is carried out with an apparatus comprising heating elements whose form is adapted to the anatomy of the areas to be depilated, in a envelope coated with flexible plastic material.

15. A hair destabilizing and atrophying compositions, characterized in that it is used before depilation and is under the form of a composition such as applied in the method defined in anyone of claims 1 to 13.

16. The process for elaborating a composition according to claim 15, comprising adding camphor to a mixture of essential oils, followed by the addition of the obtained mixture to a vegetal oil, then emulsifying the whole with alcohol at 90°, adding a sedative or softener, then incorporating the emulsion to the gel, while mixing for obtaining an homogeneous composition.

17. Ensemble for destabilizing, before depilation, hair at their roots, and atrophying the fresh growth elements, comprising a composition according to claim 15 and an equipment having heating elements whose form is adapted to the anatomy of the areas to be depilated, enabling the action of said composition to be increased by bringing it, after application to the skin, to a temperature of 25 to 50°C.

## Patentansprüche

1. Verfahren zur kosmetischen Behandlung zum Zweck einer endgültigen Epilation, dadurch gekennzeichnet, daß vor der Epilation eine haardestabilisierende und -atrophierende Zusammensetzung in Form eines essentielle Öle umfassenden Kontaktgels auf den zu epilierenden Bereich aufgebracht und diese Zusammensetzung anschließend auf eine Temperatur von 25 bis 50°C erhitzt wird.

2. Verfahren nach Anspruch 1, dadurch gekennzeichnet, daß die angewandte Zusammensetzung aus einem Gemisch von essentiellen Ölen und pflanzlichem Öl erhalten wird.

3. Verfahren nach Anspruch 2, dadurch gekennzeichnet, daß die angewandte Zusammensetzung darüber hinaus Campher, Alkohol, ein beruhigendes oder weichmachendes Mittel pflanzlichen Ursprungs enthält.

4. Verfahren nach einem der Ansprüche 1 oder 3, dadurch gekennzeichnet, daß die essentiellen Öle unter Birkenöl, Zitronenöl, Zypressenöl, Geranienöl, Lavendelöl, Pinienöl und Verbenenöl ausgewählt sind.

5. Verfahren nach einem der Ansprüche 2 bis 3, dadurch gekennzeichnet, daß das pflanzliche Öl Süßmandelöl ist.

6. Verfahren nach einem der Ansprüche 3 bis 5, dadurch gekennzeichnet, daß das beruhigende oder weichmachende Mittel Azulen ist.

7. Verfahren nach einem der Ansprüche 1 bis 6, dadurch gekennzeichnet, daß das Gel aus Hydroxyethylcellulose, Propylenglykol, p-Methylhydroxybenzoat und p-Propylhydroxybenzoat und Natriumsulfat hergestellt wird.

8. Verfahren nach einem der Ansprüche 1 bis 7, dadurch gekennzeichnet, daß die Zusammensetzung ein Gemisch essentieller Öle nach Anspruch 4 in den folgenden Verhältnissen umfaßt:
Birkenöl 5 bis 30 Tropfen,
d.s. 0,11 bis 0,64 ml (47 Tropfen pro ml);
Zitronenöl 15 bis 120 Tropfen, d.s. 0,41 bis 3,24 ml (37 Tropfen pro ml);
Zypressenöl 5 bis 30 Tropfen,
d.s. 0,12 bis 0,70 ml (43 Tropfen pro ml);
Geraniumöl 5 bis 50 Tropfen,
d.s. 0,15 bis 1,25 ml (40 Tropfen pro ml);
Lavendelöl 5 bis 120 Tropfen,
d.s. 0,12 bis 2,86 ml (42 Tropfen pro ml);
Pinienöl 5 bis 30 Tropfen,
d.s. 0,11 bis 0,65 ml (46 Tropfen pro ml);
Verbenenöl 10 bis 80 Tropfen
d.s. 0,23 bis 1,86 ml (43 Tropfen pro ml).

9. Verfahren nach Anspruch 8, dadurch gekennzeichnet, daß die Zusammensetzung darüber hinaus 10 bis 200 Tropfen Campher, d.s. 0,22 bis 4,44 ml (45 Tropfen pro ml), umfaßt.

10. Verfahren nach Anspruch 8 oder 9, dadurch gekennzeichnet, daß die Zusammensetzung darüber hinaus 2 bis 20 ml pflanzliches Öl umfaßt.

11. Verfahren nach einem der Ansprüche 8 bis 10, dadurch gekennzeichnet, daß die Zusammensetzung darüber hinaus 2,5 bis 30 ml Alkohol von 90° umfaßt.

12. Verfahren nach einem der Ansprüche 8 bis 11, dadurch gekennzeichnet, daß die Zusammensetzung darüber hinaus 2 bis 25 ml eines beruhigenden oder weichmachenden Mittels umfaßt.

13. Verfahren nach einem der Ansprüche 8 bis 12, dadurch gekennzeichnet, daß die Zusammensetzung ein Gel der folgenden Zusammensetzung umfaßt:
| | |
|---|---|
| Hydroxyethylcellulose | 2,5 g |
| Propylenglycol | 4 g |
| p-Methylhydroxybenzoat und p-Propylhydroxybenzoat | 0,15 g |
| Natriumsulfat | 1 g |
| Patentblau V | 2 ppm |
| Gereinigtes Wasser q.s.p. | 100 g |

14. Verfahren nach einem der Ansprüche 1 bis 13, dadurch gekennzeichnet, daß das Erhitzen mit Hilfe einer Vorrichtung ausgeführt wird, welche Heizelemente enthält, deren Form an die Anatomie der zu epilierenden Bereiche angepaßt ist, die von einer Hülle aus geschmeidigen Kunststoffmaterial umgeben sind.

15. Haardestabilisierende und -atrophierende Zusammensetzung, dadurch gekennzeichnet, daß sie vor der Epilierung anwendbar ist und in Form einer Zusammensetzung vorliegt, welche gemäß dem Verfahren angewandt wird, wie es in einem der Ansprüche 1 bis 13 beschrieben ist.

16. Verfahren zur Herstellung einer Zusammensetzung nach Anspruch 15, dadurch gekennzeichnet, daß es die Zugabe von Campher zu einem Gemisch essentieller Öle, gefolgt von der Zugabe des erhaltenen Gemisches zu eines pflanzlichen Öl, die anschließende Emulgierung des Ganzen mit einem Alkohol von 90°, die Zugabe eines beruhigenden oder weichmachenden Mittels und anschließend die Einverleibung der Emulsion in ein Gel unter Rühren, um eine homogone Zusammensetzung zu erhalten, umfaßt.

17. Zusammenstellung zur Destabilisierung von Haaren an ihrer Wurzel vor der Epilation und zur Atrophierung der Wachstumselemente, dadurch gekennzeichnet, daß die Zusammenstellung eine Zusammensetzung nach Anspruch 15 und eine Vorrichtung umfaßt, welche Heizelemente aufweist, deren Form an die Anatomie der zu epilierenden Gebiete angepaßt ist, welche die Beschleunigung der Wirkung dieser Zusammensetzung nach der Aufbringung auf die Haut durch ein Erhitzen auf eine Temperatur von 25 bis 50°C ermöglicht.
